# EUROPEAN PATENT APPLICATION

(11) **EP 4 806 369 A1**
(43) Date of publication of application: **16.09.2026**
(21) Application number: 26163476.0
(22) Date of filing: 10.03.2026
(51) Int. Cl.: A61B 8/08, A61B 8/12, A61B 8/14, A61B 8/00

(54) **SYSTEMS AND METHODS FOR LOCATING A MEDICAL DEVICE**

(30) Priority: 12.03.2025 US 202563770666 P
(71) Applicant: Boston Scientific Scimed, Inc., Maple Grove, Minnesota 55311 (US)
(72) Inventor: Li, Yan, Maple Grove, MN, 55311-1566 (US); Li, Qian, Maple Grove, MN, 55311-1566 (US); Li, Wenguang, Maple Grove, MN, 55311-1566 (US); Stepkowski, Marc J., Maple Grove, MN, 55311-1566 (US)
(74) Representative: Vossius & Partner Patentanwälte Rechtsanwälte mbB

(57) **Abstract**

Methods and systems are provided for locating a medical device within vasculature. Methods and systems may include receiving a series of intravascular image frames captured along a portion of the vessel; extracting a plurality of longitudinal images from the series of intravascular image frames, each one of the plurality of longitudinal images sampled at a different angle. Methods and systems may include condensing each of the plurality of longitudinal images into corresponding longitudinal intensity values; averaging the longitudinal intensity values for each of the plurality of longitudinal images; deriving a series of intensity variation values based in part on the average longitudinal intensity values for each of the plurality of longitudinal images; identifying a longitudinal location along the portion of the vessel based on the series of intensity variation values; and outputting an indication of a frame of the series of intravascular image frames corresponding to the longitudinal location.

## Description

### CROSS-REFERENCE TO RELATED APPLICATION

This application claims the benefit of U.S. Patent Application Serial No. 63/770,666, filed March 12, 2025, entitled "SYSTEMS AND METHODS FOR LOCATING A MEDICAL DEVICE", which is incorporated by reference herein in its entirety.

### TECHNICAL FIELD

The present disclosure generally relates to intravascular ultrasound (IVUS) imaging methods and systems. More particularly, but not exclusively, the present disclosure relates to detecting a guide catheter or other like device within the vasculature of a subject or patient during IVUS imaging.

### BACKGROUND

Ultrasound devices insertable into patients have proven diagnostic capabilities for a variety of diseases and disorders. For example, intravascular ultrasound (IVUS) imaging systems have been used as an imaging modality for diagnosing blocked blood vessels and providing information to aid medical practitioners in selecting and placing stents, selecting sites for an atherectomy procedure, or the like.

IVUS imaging systems include a control module (with a pulse generator, an image acquisition and processing components, and a monitor), a catheter, and a transducer disposed in the catheter. The transducer-containing catheter is positioned in a lumen or cavity within, or in proximity to, a region to be imaged, such as a blood vessel wall or patient tissue in proximity to a blood vessel wall. The pulse generator in the control module generates electrical pulses that are delivered to the transducer and transformed to acoustic pulses that are transmitted through patient tissue. The patient tissue (or other structure) reflects the acoustic pulses and the reflected pulses are absorbed by the transducer and transformed to electric pulses. The transformed electric pulses are delivered to the image acquisition and processing components and converted into images displayable on the monitor.

CT coronary angiography (CTA or CCTA) is the use of CT angiography to assess the coronary arteries of the heart via an extravascular image. Typically, a patient receives an intravenous injection of contrast agent and then the heart is scanned using a high-speed CT scanner. CTA and IVUS are often used in conjunction with each other. For example, a physician will use the CTA and the IVUS to assess the extent of an occlusion (or occlusions) in the coronary arteries, usually to diagnose coronary artery disease.

To aid physicians in reviewing these images, they are often co-registered to each other. For example, each image in a series of IVUS images can be mapped, or co-located, to a position of the vessel represented in the CTA image. Co-registration of angiography and IVUS images is a transformative technique that enhances the assessment of coronary artery disease by offering a comprehensive understanding of plaque composition, severity, and vessel anatomy. This advancement significantly improves the precision of stent placement, treatment strategizing, and lesion monitoring.

However, currently, co-registration is a manual process which takes time and input from the physician. Therefore, many unmet needs exist in the art to provide improved co-registration techniques and workflows.

### BRIEF SUMMARY

This Summary is provided to introduce a selection of concepts in a simplified form that are further described below in the Detailed Description. This Summary is not intended to necessarily identify key features or essential features of the claimed subject matter, nor is it intended as an aid in determining the scope of the claimed subject matter.

In general, the present disclosure provides workflows, systems, graphical user interfaces, methods and processes to co-register IVUS images with external images, such as, an angiogram image. The present disclosure also may provide methods and systems for locating and identifying a medical device, such as a guide catheter or other like device, during an imaging procedure. In other non-limiting examples, the present disclosure may provide methods and systems for locating and identifying a medical device, such as a guide catheter or other like device, using a single imaging modality during an imaging procedure.

In some implementations, the present disclosure may be embodied as a method, for example a computer-implemented method of locating a guide catheter within vasculature, the method including: receiving a series of intravascular image frames captured along a portion of a vessel, extracting a plurality of longitudinal images from the series of intravascular image frames, whereby each one of the plurality of longitudinal images is sampled at a different angle. Methods of this and other examples may further include: condensing each of the plurality of longitudinal images into corresponding longitudinal intensity values, averaging the longitudinal intensity values for each of the plurality of longitudinal images, deriving a series of intensity variation values based in part on the average values for each of the plurality of longitudinal images, identifying a longitudinal location along the portion of the vessel based on the series of intensity variation values, and outputting an indication of a frame of the series of intravascular image frames corresponding to the longitudinal location.

Alternatively, or additionally to the methods disclosed herein, extracting the plurality of longitudinal images from the series of intravascular image frames may include: sampling the series of intravascular image frames at a plurality of angles, and generating, for each one of the plurality of angles, a longitudinal image of the plurality of longitudinal images, whereby the longitudinal image is representative of the frames of the plurality of intravascular image frames associated with the one of the plurality of angles.

Alternatively, or additionally to the methods disclosed herein, condensing each of the plurality of longitudinal images into corresponding longitudinal intensity values may include summing the intensity at each longitudinal location.

Alternatively, or additionally to the methods disclosed herein, deriving a series of intensity variation values may include a sliding window translatable along a longitudinal direction.

Alternatively, or additionally to the methods disclosed herein, the frame of the series of intravascular image frames may depict a guide catheter.

Alternatively, or additionally to the methods disclosed herein, the frame of the series of intravascular image frames indicates a diameter of a guide catheter.

Alternatively, or additionally to the methods disclosed herein, extracting a plurality of longitudinal images may include extracting four or more longitudinal images.

Alternatively, or additionally to the methods disclosed herein, the series of intravascular image frames may be captured using intravascular ultrasound (IVUS) or optical coherence tomography (OCT).

In some implementations, the present disclosure may be embodied as an apparatus and/or system for an intravascular ultrasound (IVUS) imaging system, and may include, for example: a display, an interface configured to couple to an IVUS imaging modality, a processor coupled to the interface and the display, and a memory device including instructions, which when executed by the processor, cause the IVUS imaging system to: receive a series of intravascular image frames captured along a portion of a vessel. In this and other examples, the processor may also cause the IVUS imaging system to: extract a plurality of longitudinal images from the series of intravascular image frames, whereby each one of the plurality of longitudinal images is sampled at a different angle; condense each of the plurality of longitudinal images into corresponding longitudinal intensity values; average the longitudinal intensity values for each of the plurality of longitudinal images; derive a series of intensity variation values based in part on the average longitudinal intensity values for each of the plurality of longitudinal images; identify a longitudinal location along the portion of the vessel based on the series of intensity variation values; and output an indication of a frame of the series of intravascular image frames corresponding to the longitudinal location.

Alternatively, or additionally to the apparatuses and systems disclosed herein, the extracted plurality of longitudinal images from the series of intravascular image frames may include: a series of intravascular image frames sampled at a plurality of angles, and for each one of the plurality of angles, a generated longitudinal image of the plurality of longitudinal images, whereby the longitudinal image is representative of the frames of the plurality of intravascular image frames associated with the one of the plurality of angles.

Alternatively, or additionally to the apparatuses and systems disclosed herein, each of the plurality of images may be condensed into corresponding longitudinal intensity values by summing the intensity at each longitudinal location.

Alternatively, or additionally to the apparatuses and systems disclosed herein, the frame of the series of intravascular image frames may depict a guide catheter.

Alternatively, or additionally to the apparatuses and systems disclosed herein, the frame of the series of intravascular image frames may indicate a diameter of a guide catheter.

Alternatively, or additionally to the apparatuses and systems disclosed herein, the extraction of the plurality of longitudinal images may include extracting four or more longitudinal images.

Alternatively, or additionally to the apparatuses and systems disclosed herein, the series of intravascular image frames may be captured using intravascular ultrasound (IVUS) or optical coherence tomography (OCT).

In some implementations, the present disclosure may be embodied as a non-transitory computer-readable storage medium, and may include, for example: instructions, that when executed by a computer, cause the computer to: receive a series of intravascular image frames captured along a portion of a vessel. In this and other examples, the instructions, that when executed by a computer, may cause the computer to: extract a plurality of longitudinal images from the series of intravascular image frames, whereby each one of the plurality of images may be sampled at a different angle; condense each of the plurality of longitudinal images into corresponding longitudinal intensity values; average the longitudinal intensity values for each of the plurality of longitudinal images; derive a series of intensity variation values based in part on the average longitudinal intensity values for each of the plurality of longitudinal images; identify a longitudinal location along the portion of the vessel based on the series of intensity variation values; and output an indication of a frame of the series of intravascular image frames corresponding to the longitudinal location.

Alternatively, or additionally to the non-transitory computer-readable media disclosed herein, the frame of the series of intravascular image frames may depict a guide catheter.

Alternatively, or additionally to the non-transitory computer-readable media disclosed herein, the frame of the series of intravascular image frames may indicate a diameter of a guide catheter.

Alternatively, or additionally to the non-transitory computer-readable media disclosed herein, the extraction of the plurality of longitudinal images from the series of intravascular image frames may include: sampling the series of intravascular image frames at a plurality of angles, and generating, for each one of the plurality of angles, a longitudinal image of the plurality of longitudinal images, whereby the longitudinal image is representative of the frames of the plurality of intravascular image frames associated with the one of the plurality of angles.

Alternatively, or additionally to the non-transitory computer-readable media disclosed herein, whereby the condensed plurality of longitudinal images are condensed into corresponding longitudinal intensity values by summing the intensity at each longitudinal location.

Alternatively, or additionally to the non-transitory computer-readable media disclosed herein, the derived series of intensity values may be derived via a sliding window translatable along a longitudinal direction.

Alternatively, or additionally to the non-transitory computer-readable media disclosed herein, the extracted plurality of longitudinal images may include extracting four or more longitudinal images.

Alternatively, or additionally to the non-transitory computer-readable media disclosed herein, the series of intravascular image frames may be captured using intravascular ultrasound (IVUS) or optical coherence tomography (OCT).

### BRIEF DESCRIPTION OF THE SEVERAL VIEWS OF THE DRAWINGS

To easily identify the discussion of any element or act, the most significant digit or digits in a reference number refer to the figure number in which that element is first introduced.
FIG. 1 illustrates a multi-modality medical device locating system, in accordance with at least one embodiment.
FIG. 2 illustrates a routine for locating a medical device and deriving associated information in accordance with at least one embodiment.
FIG. 3A shows a series of longitudinal images extracted from one or more imaging angles in accordance with at least one embodiment.
FIG. 3B shows a series of condensed longitudinal images condensed into corresponding intensity plots in accordance with at least one embodiment.
FIG. 3C shows an average of the plots from FIG. 3B, in accordance with at least one embodiment.
FIG. 3D shows the calculation of intensity variation via a sliding window along a longitudinal direction, in accordance with at least one embodiment.
FIG. 4A illustrates an example vascular imaging system, in accordance with at least one embodiment.
FIG. 4B illustrates an enlarged view of an example intravascular imaging device in accordance with at least one embodiment.
FIG. 5 illustrates a diagrammatic representation of a machine in the form of a computer system within which a set of instructions may be executed for causing the machine to perform any one or more of the methodologies discussed herein, according to at least one embodiment.

### DETAILED DESCRIPTION

As noted above, the present disclosure provides systems and techniques to locate, detect, and identify a medical device (such as a guidewire, guide catheter, or the like) from different imaging modalities (e.g., IVUS, OCT and angiography). It is noted that although the present disclosure uses examples and describes techniques with reference to IVUS, OCT, and angiography, any suitable extravascular imaging modality and/or intravascular imaging modality can be utilized. Further, the present disclosure provides detection and location of medical devices and their associated information (e.g., exact location, size, diameter, orientation, etc.) from the captured and/or obtained imaging data of one or more imaging modalities.

Generally, IVUS image frames are multi-dimensional multivariate images comprising indications of the vessel type, a lesion in the vessel, the lesion type, stent detection, the lumen border, the lumen dimensions, the minimum lumen area (MLA), the media border (e.g., a media border for media within the blood vessel), the media dimensions, the calcification angle/arc, the calcification coverage, combinations thereof, and/or the like. In combination with the principles of the present disclosure, IVUS image frames, optical coherence tomography (OCT) image frames, or the like may be utilized to provide location information and other associated information (i.e., data) regarding a medical device (such as a guide catheter) used during an imaging procedure.

Suitable IVUS imaging systems include, but are not limited to, one or more transducers disposed on a distal end of a catheter configured and arranged for percutaneous insertion into a patient. Examples of IVUS imaging systems with catheters are found in, for example, U.S. Pat. Nos. 7,246,959; 7,306,561; and 6,945,938; as well as U.S. Patent Application Publication Numbers 2006/0100522; 2006/0106320; 2006/0173350; 2006/0253028; 2007/0016054; and 2007/0038111; all of which are incorporated herein by reference.

In optical coherence tomography (OCT) imaging, images are created by mapping the scattering properties of tissue subject to imaging. This technique incorporates focusing optics and a reference to create a coherence interference signal. Additionally, chirped pulse interferometry, which is an extension of OCT, can also be utilized which entails imaging using oppositely-chirped laser pulses in order to avoid dispersion (providing more depth penetration of pulses). Another known imaging technique is optical coherence elastography (OCE). In optical coherence elastography, images are created by mapping the mechanical properties of a tissue. This technique incorporates an applied force (balloon or possible acoustic/mechanical forces from pulsed laser) combined with an OCT imaging system. OCE data and images can be used to identify different plaque types before and after an intravascular procedure in addition to other capabilities known in the art.

As noted, the present disclosure relates to intravascular imaging modalities (e.g., IVUS, OCT, etc.) that capture images and lumens (e.g., vessels) of patients and to processing these images and to adapting features from one modality and integrating them into another modality. As such, an example intravascular imaging system, patient vessel, and series of intravascular images are described below. It is noted that the example system is described as an IVUS system. However, it will be appreciated that the described system could be an OCT system (or other intravascular imaging modality) and/or a combined non-IVUS/IVUS system without departing from the scope of the disclosure. It can be further appreciated that the principles and aims of the present disclosure may be achieved through a single imaging modality, including but not limited to IVUS imaging, OCT imaging, near-infrared spectroscopy (NIRS) imaging, near-infrared fluorescence (NIRF) imaging, photoacoustic imaging, fluorescence-lifetime imaging, combinations thereof and/or other like internal and/or endoluminal imaging processes, procedures and techniques.

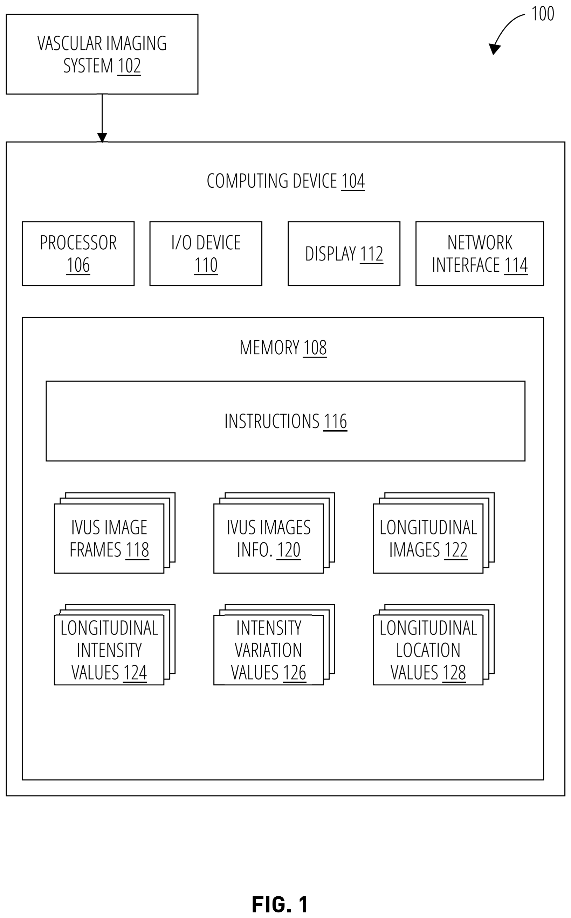

FIG. 1 illustrates a multi-modality medical device locating system, in accordance with at least one embodiment of the present disclosure. In general, medical device locating system 100 is a system configured to detect and indicate a medical device or other like structure from one or more of intravascular (e.g., IVUS) image frames 118. The detected locations can be used to co-register the intravascular image frames with one or more extravascular images (e.g., angiography).

Medical device locating system 100 can be configured to receive a series of image frames 118 captured along a portion of a vessel within a patient or the patient's vasculature via a vascular imaging system 102, and the image frames 118 may be captured from one or more of intravascular imaging modalities or like techniques as will be described further herein.

Vascular imaging system 102 may be communicable with one or more computing devices, such as computing device 104. Vascular imaging system 102 may communicate with one or more computing devices wirelessly, wired, and/or via one or more networks and/or network and/or communication modalities. In this and other examples, computing device 104 may include and/or be incorporated and/or communicable with a processor 106, an input/output (I/O) device 110, a display 112, and a network interface 114. It is also contemplated that a plurality of these aforementioned components and elements may be provided. Computing device 104 may also include a memory 108 which includes instructions 116 and may further include storage for IVUS image frames 118, IVUS image information 120, longitudinal images 122 (i.e., one or more or a series of longitudinal images), longitudinal intensity values 124, intensity variation values 126, and longitudinal location values 128, and additional elements and components, all of which will be described further herein.

Alternatively, or additionally, the system 100 depicted in FIG. 1 and applicable to other examples may be a single-modality medical device locating system. In this and other examples, the singular modality may be intravascular ultrasound imaging (IVUS), optical coherence tomography (OCT), or any like or similar imaging modality. In this and other examples, the singular modality of the single-modality system 100 may be an internal imaging modality, such as IVUS or OCT, but in other non-limiting examples, the singular modality may be an external imaging modality, such as angiography or the like.

In any of the above examples, the system 100, and particularly the computing device 104, is configured to receive IVUS image frames 118. As described, IVUS imaging systems (e.g., vascular imaging system 102, or the like) are used to capture a series of images or a "recording" of a vessel, a portion of a vessel, and/or a portion of patient or subject vasculature. For example, an IVUS catheter (described further herein) is inserted into a vessel and a recording, or a series of IVUS images, is captured as the catheter is pulled back from a distal end of an IVUS pullback path to a proximal end of an IVUS pullback path, for example, during an IVUS pullback operation. The catheter can be pulled back manually or automatically (e.g., under control of one or more drive units, or the like) as will be described further herein.

To that end, the vascular imaging system 102 may include an intravascular imaging device such as an imaging catheter which will be described further herein. The imaging catheter may be configured to be inserted within a patient or subject so that its distal end, including a diagnostic assembly or probe (e.g., an IVUS probe or the like), is in the vicinity of a desired imaging location of a blood vessel, blood vessel branch, blood vessel portion, or a section and/or selection of patient or subject vasculature. A radiopaque material or marker (not shown) may be additionally or alternatively provided and located on or near the probe to provide indicia of a current location of the probe in a radiological and/or angiographic image. As will be appreciated by those of ordinary skill in the art, the intravascular imaging device (e.g., IVUS catheter, or the like) may be inserted into the patient's vessel over a guidewire.

As outlined above, IVUS (or other intravascular image modality) image frames are often co-registered with an extravascular image frame as part of a percutaneous intervention (PCI) procedure. In conventional co-registration procedures, a physician is required to mark the "start" and "end" locations of the IVUS image frames on the extravascular image. Unfortunately, this may introduce errors or inconsistencies into the co-registration procedure and also requires receiving input from a user (e.g., physician) and further adds time to the co-registration process.

Accordingly, the present disclosure provides to identify a medical device (e.g., a guidewire or guide catheter) in one or more of the IVUS image frames 118. Subsequently, the location of the medical device can be used to identify the end location of the IVUS image frames on an extravascular image as part of a co-registration process in which the IVUS image frames 118 are co-registered to an extravascular image.

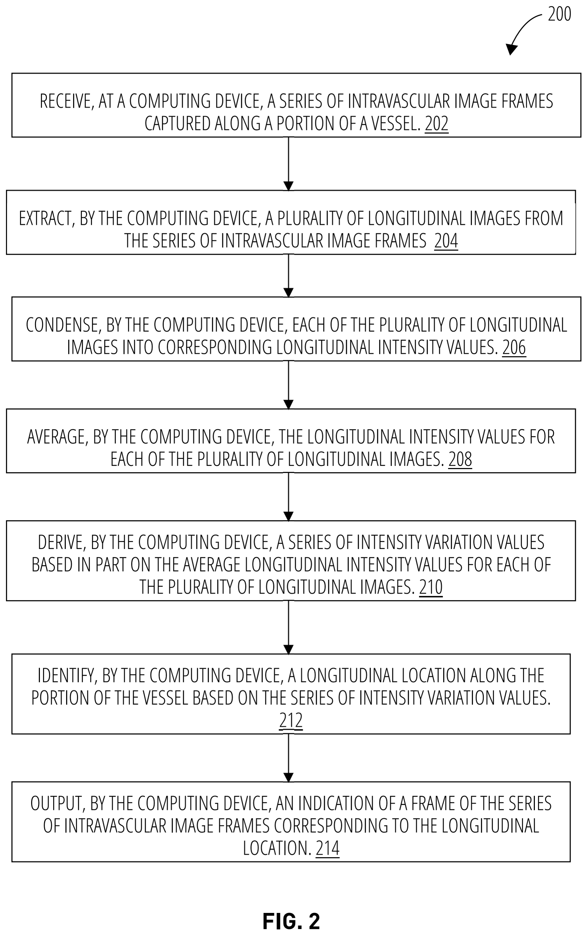

FIG. 2 illustrates a routine or workflow for locating a medical device and deriving associated information in accordance with at least one embodiment of the present disclosure. Routine 200 can begin at block 202 "receive, at a computing device, a series of intravascular image frames captured along a portion of a vessel." For example, processor 106 can execute instructions 118 to receive a series of intravascular image frames captured along a portion of a vessel, along a section of a vessel, along one or more vessels, or along a selection of vasculature. Methods and systems of the present disclosure may utilize an imaging system, such as vascular imaging system 102 (in coordination with one or more computing devices) to execute instructions 118 to receive a series of intravascular image frames captured and/or obtained along a portion of a vessel, along one or more vessels, or along a selection of vasculature from one or more imaging devices disclosed herein.

Continuing to block 204 "extract, by the computing device, a plurality of longitudinal images from the series of intravascular image frames." For example, processor 106 can execute instructions 118 to extract a plurality of longitudinal images from the series of intravascular image frames. Methods and systems of the present disclosure may utilize an imaging system, such as vascular imaging system 102 (in coordination with one or more computing devices) to execute instructions 118 to extract a plurality of longitudinal images (i.e., images obtained along the longitudinal axis of a blood vessel, a portion of a blood vessel, and/or a portion or selection of vasculature) from the series of intravascular image frames captured and/or obtained by the vascular imaging system 102. In some examples, longitudinal images 122 may be extracted from the IVUS image frames 118 where each one of longitudinal images 122 corresponds to a different angle. In other words, each group of longitudinal images 122 may correspond to a particular imaging angle or a range of angles. For example, as outlined above, the IVUS image frames 118 are captured by a rotating ultrasound transducer. Frames from the IVUS image frames 118 corresponding to a particular angle of rotation at which the frame is captured (e.g., 0 degrees, 60 degrees, 90 degrees, etc.) or range of angles of rotation at which the frame is captured (e.g., 0 degrees to 59 degrees, 60 degrees to 89 degrees, 90 degrees, to 119 degrees, etc.) may be extracted and grouped together as longitudinal images 122.

Continuing to block 206 "condense, by the computing device, each of the plurality of longitudinal images into corresponding longitudinal intensity values." For example, processor 106 can execute instructions 118 to condense each of the plurality of longitudinal images into corresponding longitudinal intensity values. In other words, and by non-limiting example, processor 106 can execute instructions 118 to convert each of the plurality of longitudinal images into converted longitudinal intensity values that reflect the intensity (i.e., brightness) of each of the plurality of longitudinal images into corresponding longitudinal intensity values. Methods and systems of the present disclosure may utilize an imaging system, such as vascular imaging system 102 (in coordination with one or more computing devices) to execute instructions 118 to condense each of the plurality of longitudinal images into corresponding longitudinal intensity values, in other words, vascular imaging system 102 (in coordination with one or more computing devices) may convert a plurality of longitudinal images into corresponding longitudinal intensity values depicting the intensity (i.e., brightness) of captured images along a portion of a vessel and/or along a portion of one or more vessels and/or along a selection of vasculature.

Continuing to block 208 "average, by the computing device, the longitudinal intensity values for each of the plurality of longitudinal images." For example, processor 106 can execute instructions 118 to average the longitudinal intensity values for each of the plurality of longitudinal images. Methods and systems of the present disclosure may utilize an imaging system, such as vascular imaging system 102 (in coordination with one or more computing devices) to execute instructions 118 to average the longitudinal intensity values for each of the plurality of longitudinal images.

Continuing to block 210 "derive, by the computing device, a series of intensity variation values based in part on the average longitudinal intensity values for each of the plurality of longitudinal images." For example, processor 106 can execute instructions 118 to derive a series of intensity variation values based in part on the average longitudinal intensity values for each of the plurality of longitudinal images. Methods and systems of the present disclosure may utilize an imaging system, such as vascular imaging system 102 (in coordination with one or more computing devices) to execute instructions 118 to derive a series of intensity variation values based in part on the average longitudinal intensity values for each of the plurality of longitudinal images.

Continuing to block 212 "identify, by the computing device, a longitudinal location along the portion of the vessel based on the series of intensity variation values." For example, processor 106 can execute instructions 118 to identify a longitudinal location (e.g., a location along the longitudinal axis of the vessel represented by the captured image) along the portion of the vessel based on the series of intensity variation values. Methods and systems of the present disclosure may utilize an imaging system, such as vascular imaging system 102 (in coordination with one or more computing devices) to execute instructions 118 to identify a longitudinal location along the portion of the vessel based on the series of intensity variation values.

Continuing to block 214 "output, by the computing device, an indication of a frame of the series of intravascular image frames corresponding to the longitudinal location." For example, processor 106 can execute instructions 118 to output an indication of a frame of the series of intravascular image frames corresponding to the longitudinal location. Methods and systems of the present disclosure may utilize an imaging system, such as vascular imaging system 102 (in coordination with one or more computing devices) to execute instructions 118 to output an indication of a frame of the series of intravascular image frames corresponding to the longitudinal location. In this and other examples, the indication of a frame may be output to a display device and/or user interface such as a graphical user interface (GUI). Other display outputs are also contemplated, including but not limited to an LCD display, an LED display, an OLED display, a semi-transparent display, a head-worn display, a portable display, a remote display or other like displays and viewing modalities.

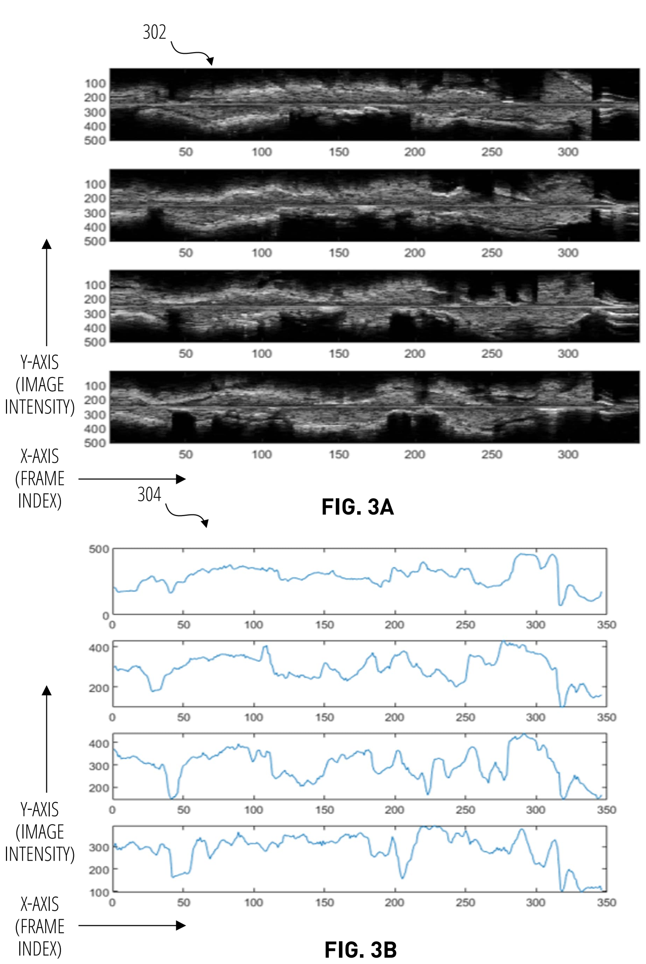

FIG. 3A shows a series of longitudinal images 302 extracted from one or more imaging angles in accordance with at least one embodiment of the present disclosure. Along the X-axis, frame index is depicted. In other words, the X-axis values shown in corresponding FIG. 3A represent frame index values (i.e., the indexed number of the frame within the frame index). Along the Y-axis, image intensity values are plotted against their respective frame index values. In this and other examples, one or more longitudinal images 302 may be extracted from an IVUS imaging system or other like imaging modality, such as optical coherence tomography (OCT) and/or optical coherence elastography (OCE). In this and other examples, two or more longitudinal images may be extracted from an IVUS imaging system or other like imaging modality. In other non-limiting examples, three or more longitudinal images may be extracted. In yet other non-limiting examples, four or more longitudinal images may be extracted.

FIG. 3B shows a series of condensed longitudinal images 304 condensed into corresponding intensity plots in accordance with at least one embodiment of the present disclosure. Along the X-axis, frame index is depicted. In other words, the X-axis values shown in corresponding FIG. 3B represent frame index values (i.e., the indexed number of the frame within the frame index). Along the Y-axis, image intensity values are plotted against their respective frame index values. At this step, a series of condensed longitudinal images 304 are converted into corresponding plots reflecting a longitudinal position of at least a portion of a vessel of a patient or subject vasculature and are plotted in units of intensity (i.e., brightness). In other words, the brightness of the converted image is synonymous with its intensity.

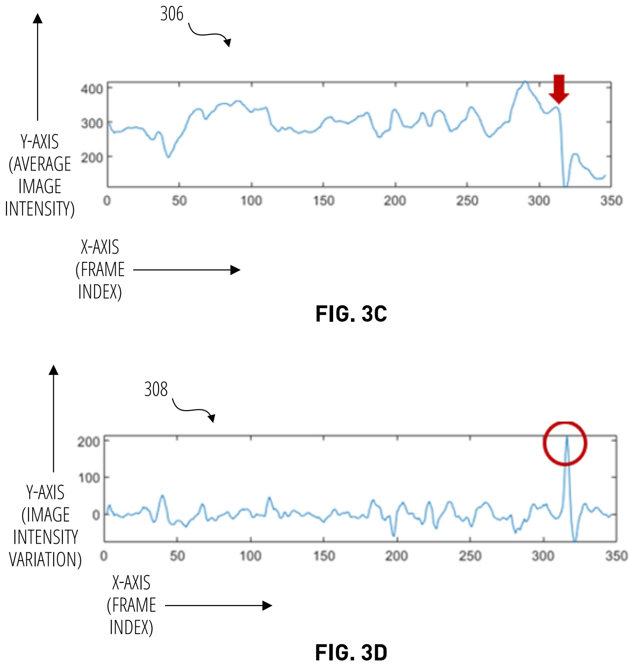

FIG. 3C shows an average of the plots 306 from FIG. 3B, in accordance with at least one embodiment of the present disclosure. Along the X-axis, frame index is depicted. In other words, the X-axis values shown in corresponding FIG. 3C represent frame index values (i.e., the indexed number of the frame within the frame index). Along the Y-axis, image intensity values are plotted against their respective frame index values. The average of the plots 306 may be derived and/or determined from all longitudinal images, resulting in the plot 306 shown in FIG. 3C, for example. The arrow shown in FIG. 3C indicates the reveal of an inflection point of intensity variation (i.e., where the brightness of the image acutely inflects and the corresponding plotted data changes slope into greater (positive slope) or weaker (negative slope) intensity).

Once the plots are averaged, FIG. 3D shows the calculation of intensity variation via a sliding window along a longitudinal direction, in accordance with at least one embodiment of the present disclosure. Along the X-axis, frame index is depicted. In other words, the X-axis values shown in corresponding FIG. 3D represent frame index values (i.e., the indexed number of the frame within the frame index). Along the Y-axis, image intensity variation values are plotted against their respective frame index values (indicated along the X-axis). As shown in FIG. 3D, the intensity variation values display the variation of the average intensity values calculated from the average of the plots shown in FIG. 3C.

The circle shown in FIG. 3D indicates the point of maximum intensity variation, which confirms the location of a medical device such as a guide catheter, as the entry and/or presence of a guide catheter (or other like device) causes the brightness of the image to acutely plummet, thus revealing the IVUS (or another like modality) image frame to which at least a portion of the guide catheter (or another like device) resides. It can be appreciated that the IVUS image frame, and/or IVUS image frame number provides data and/or information that allows the medical device locating system 100 to infer the exact position of a guide catheter (or other like device), a diameter of the guide catheter (or other like device), such as an interior diameter, an exterior diameter, and/or both an interior and exterior diameter of a guide catheter or other like medical device in addition to information and/or data received from the maximum intensity variation (i.e., maximum slope inflection of intensity variation) from the intensity variation plot 306.

In this and other examples, the vascular imaging systems and medical device locating systems of the present disclosure are capable of finding imaged characteristics of imaged elements such as element borders (e.g., the border of a medical device) and may further determine measurements of imaged elements from obtained imaging data and information (e.g., IVUS image frame data and information). It can be appreciated that any imaged element may include imaged elements of medical devices including but not limited to medical devices such as a guide catheter or a guidewire. It can be further appreciated that the vascular imaging systems and medical device locating systems of the present disclosure are configured to locate, identify, and determine physical and structural features of an imaged element (such as a medical device), including but not limited to determining distances, dimensional quantities and physical and structural attributes such as diameter and dimensional measurement, as well as determining orientation, position, frame, frame number, location and appearance of imaged elements captured within obtained image frames.

The sliding window shown in FIG. 3D and applicable to other examples may include a slider (e.g., slider bar, slider widget, or other manipulatable interface component or element) that allows a user and/or practitioner of the medical device locating system 100 to advance longitudinally along the plot via a user interface such as a graphical user interface, however, this function may also be accomplished automatically by the medical device locating system 100 and outputted to any display or like device as described above.

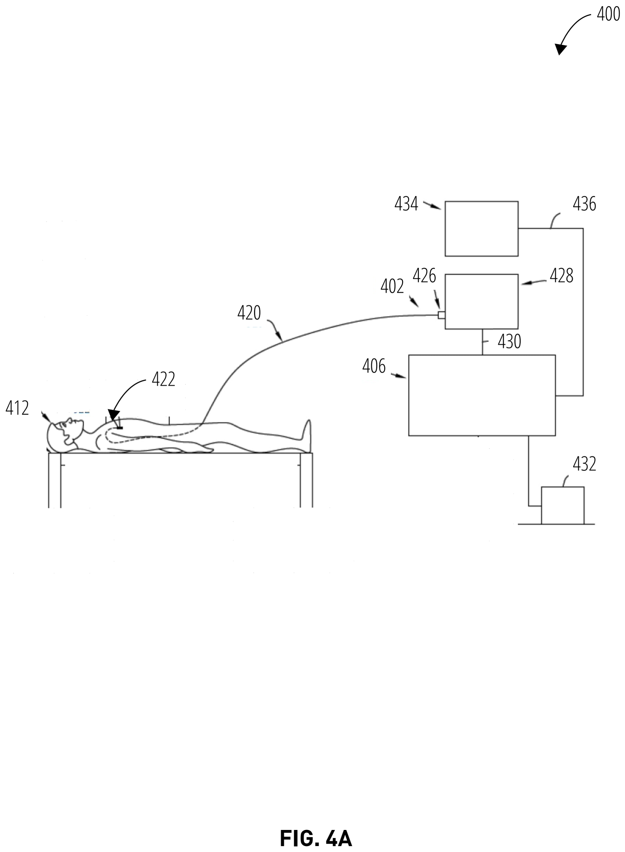

FIG. 4A illustrates an example medical device locating system 400, in accordance with at least one embodiment of the present disclosure. In this and other examples, medical device locating system 400 may include a computing device 406 connected to an image acquisition device 428 either wirelessly or through a wired connection such as transmission cable 430 or the like. Image acquisition system 428 may be further connected to an endoluminal imaging system 402 (or other like intravascular and/or internal imaging system) and may be connected via proximal connector 426 and/or any like coupling. Display 434 may also be connected to computing device 406 either wirelessly or through a wired connection such as display cable 436 or the like.

In some embodiments, computing device 406 can include user interface devices, such as, a keyboard, a mouse, a joystick, a touchscreen device (such as a smartphone or a tablet computer), a touchpad, a trackball, a voice-command interface, a brain-computer interface and/or other types of user interfaces and/or interactive interfaces that are known in the art.

The user interface can be rendered and displayed on display 434 coupled to computing device 406 via display cable 436. Although the display 434 is depicted as separate from computing device 406, in some examples the display 434 can be part of computing device 406. Alternatively, the display 434 can be remote and wireless from computing device 406. As another example, the display 434 can be part of another computing device different from computing device 406, such as, a tablet or portable computer, which can be coupled to computing device 406 via a wired or wireless connection. For some applications, the display 434 includes a head-up display and/or a head-mounted display. For some applications, the computing device 406 generates an output on a different type of visual, text, graphics, tactile, audio, and/or video output device, e.g., speakers, headphones, a smartphone, or a tablet computer. For some applications, the user interface rendered on display 434 acts as both an input device and an output device.

The computing device 406 can also include one or more additional output ports for transferring data to other devices. For example, the computing device 406 can include an output port to transfer data to a data archive or memory device 432. The computing device 406 can also include a user interface that includes a combination of circuitry, processing components and instructions executable by the processing components and/or circuitry to enable the image identification and vessel routing or pathfinding described herein and/or dynamic co-registration of intravascular and extravascular images using the identified vessel pathway.

Although not explicitly shown in FIG. 4A, medical device locating system 400 may include a combined internal and external imaging system 400 which may include both an endoluminal imaging system 402 (e.g., an IVUS imaging system, or the like) and an extravascular imaging system (not shown). Combined internal and external imaging system 400 may further incorporate the computing device 406, which includes additional and/or alternative circuitry, controllers, and/or processor(s) and memory and software as needed.

As shown in FIG. 4A and applicable to other examples, endoluminal imaging system 402 may include an imaging catheter 420 or another like device, including but not limited to a guide catheter, an intravascular guide catheter, an IVUS catheter or another like device. From there, imaging catheter 420 may be inserted into a patient 412 or subject by known means such as insertion into the vasculature of a patient or subject via a cannula, needle, sheath, sleeve or other like introducing device. An imaging probe 422 may be connected and/or coupled with and/or associated with imaging catheter 420 and placed within a patient or subject vasculature, including but not limited to one or more blood vessels within a subject or patient. Alternatively, or additionally, imaging probe 422 and/or imaging catheter 420 may include one or more optical imaging devices, or the like.

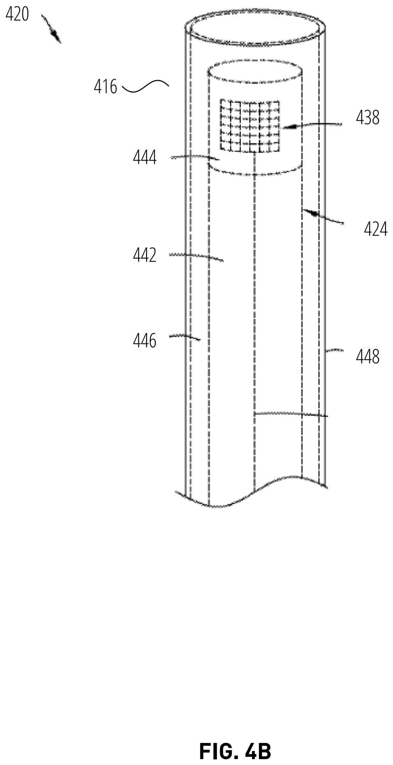

One or more of the imaging catheter 420 and imaging probe 422 may include a transducer (shown in FIG. 4B) coupled to an electric pulse generator (not shown), whereby the electric pulse generator emits electrical pulses to one or more of the imaging catheter 420 and imaging probe 422 which each may convert electrical pulses to acoustic pulses, which are then reflected off the proximate vasculature (i.e., one or more blood vessels intended to be imaged) of a patient or subject. The reflected acoustic pulses and/or waves are received by the transducer and sent to the medical device locating system 400 which produces an image from the data received by the transducer.

Imaging catheter 420 or a similar and/or like device may also include an imaging core (shown in FIG. 4B) that may be disposed within a lumen of imaging catheter 420. The imaging core may include one or more imaging devices. In at least some instances, the one or more imaging devices may include an ultrasound imaging device (e.g., an ultrasound transducer). However, other imaging devices are contemplated. For example, the one or more imaging devices may include an OCT device, a NIRS device, a NIRF device, and/or combinations thereof (e.g., including combinations that may include an IVUS imaging device).

FIG. 4B is a perspective view of an embodiment of a distal end 416 of an elongate shaft 448 of imaging catheter 420. The imaging catheter 420 may include a lumen 446 extending within a portion, a section, or within or substantially within the entirety of imaging catheter 420. An imaging core 424 may be disposed in the lumen 446. The imaging core 424 may include an imaging device 444 coupled to a distal end of a driveshaft 442 that is rotatable either manually or using a computer-controlled drive mechanism. One or more transducers 438 may be mounted to the imaging device 444 and employed to transmit and receive acoustic signals. The elongate shaft 448 may be formed from any flexible, biocompatible material suitable for insertion into a patient. Examples of suitable materials include, for example, polyethylene, polyurethane, plastic, stainless steel, spiral-cut stainless steel, nitinol, a combination of alloys, a combination of plastics, a combination of polymers and/or the like or combinations thereof.

The imaging core 424 may include and/or couple with various and/or additional and/or alternative components such as a drive shaft 442, one or more cables, a housing, and an imaging member or transducer 438 which may be coupled to the drive shaft 424 and/or housing. In at least some instances, the transducer 438 includes an ultrasound transducer. Other transducers are also contemplated. The transducer 438 may be rotatable and/or axially translatable relative to the elongate catheter shaft of imaging catheter 420. For example, the drive shaft 442 may be rotated and/or translated in order to rotate and/or translate the transducer 438 and/or one or more of the housing, the elongate shaft 448, the imaging core 424, and the imaging device 444.

The imaging core may also move longitudinally along the blood vessel within which the imaging catheter 420, an imaging probe, an IVUS probe, an IVUS catheter, a guide catheter, and/or other like device so that a plurality of cross-sectional images may be formed along a longitudinal length of the blood vessel. During an imaging procedure the one or more transducers 438 may be retracted (e.g., pulled back) along the longitudinal length of the imaging catheter 420 and/or another like device. In this and other examples, the imaging catheter 420 or other like device can include at least one telescoping section that can be retracted during pullback of the one or more transducers 438. In some instances, a drive unit (not shown) may drive the pullback of the imaging core 424 within the imaging catheter 420. The drive unit pullback distance of the imaging core 424 can be any suitable distance including, for example, at least 5 cm, 10 cm, 15 cm, 20 cm, 25 cm, or more. The imaging catheter 420 or other like device can be retracted during an imaging procedure either with or without the imaging core 424 moving longitudinally independently of the imaging catheter 420 or another like device.

A stepper motor (not shown) may optionally be used to pull back the imaging core 424. The stepper motor can pull back the imaging core 424 a short distance and stop long enough for the one or more transducers 438 to capture an image or series of images before pulling back the imaging core 424 another short distance and again capturing another image or series of images, and so on.

In non-limiting examples, the imaging catheter 420 or other like device with one or more transducers 438 mounted to the distal end of the imaging core 424 may be inserted percutaneously into a patient or subject via an accessible blood vessel, such as the femoral artery, femoral vein, or jugular vein, or at a site remote from the selected portion of the selected region, such as a blood vessel, to be imaged. The imaging catheter 420 or other like device may then be advanced through the blood vessels of the patient to the selected imaging site, such as a portion of a selected blood vessel.

An image or image frame ("frame") can be generated each time one or more acoustic signals are output to surrounding tissue and one or more corresponding echo signals are received by the imaging device 444 and transmitted to the processor 106 of the computing device 104. Alternatively, an image or image frame can be a composite of scan lines from a full or partial rotation of the imaging core 424 and/or imaging device 444. A plurality (e.g., a sequence) of frames may be acquired (i.e., captured) over time during any type of movement of the imaging device 444 and/or imaging core 424. For example, the frames can be acquired during rotation and pullback of the imaging device 444 along the target imaging location (e.g., blood vessel, portion of blood vessel, portion of vasculature, etc.). It will be understood that frames may be acquired both with or without rotation and with or without pullback of the imaging device 444 and/or imaging core 424. Moreover, it will be understood that frames may be acquired using other types of movement procedures in addition to, or in lieu of, at least one of rotation or pullback of the imaging device 444 and imaging core 424.

In some instances, when pullback (e.g., IVUS pullback) is performed, the pullback may be at a constant rate. In some instances, the imaging device 444 is pulled back at a constant rate of about 0.3-0.9 millimeters per second (mm/s) or about 0.5-0.8 mm/s. In some instances, the imaging device 444 is pulled back at a constant rate of at least 0.3 mm/s. In some instances, the imaging device 444 is pulled back at a constant rate of at least 0.4 mm/s. In some instances, the imaging device 444 is pulled back at a constant rate of at least 0.5 mm/s. In some instances, the imaging device 444 is pulled back at a constant rate of at least 0.6 mm/s. In some instances, the imaging device 444 is pulled back at a constant rate of at least 0.7 mm/s. In some instances, the imaging device 444 is pulled back at a constant rate of at least 0.8 mm/s.

In non-limiting examples, the one or more acoustic signals are output to surrounding tissue at constant intervals of time. In some instances, the one or more corresponding echo signals are received by the imaging device 444 and transmitted to the processor 106 of the computing device 104 at constant intervals of time. In some instances, the resulting frames are generated at constant or substantially constant intervals of time.

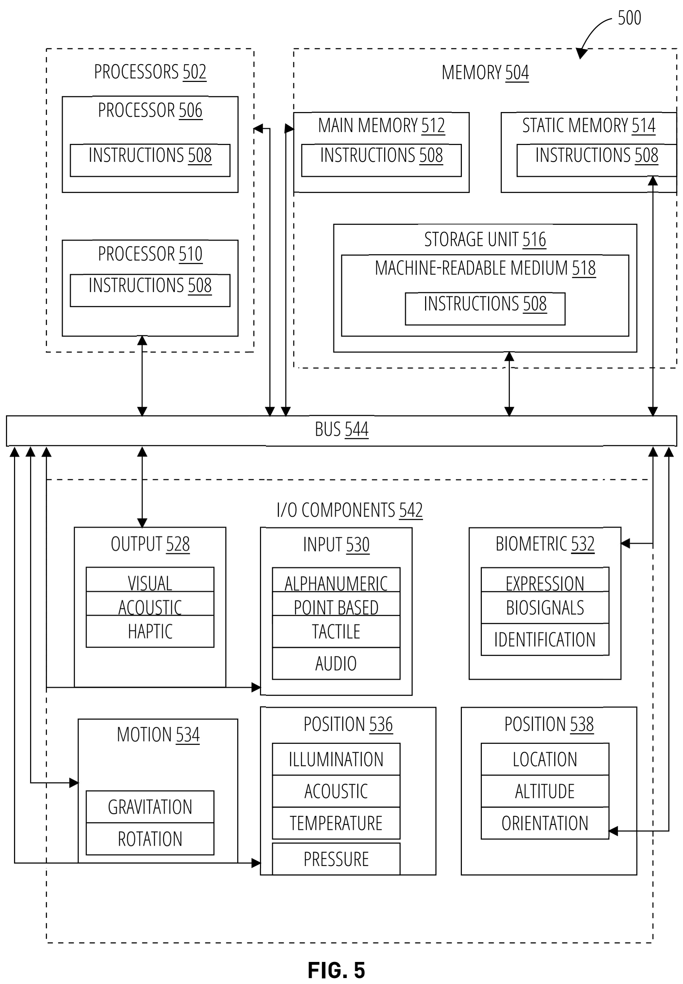

FIG. 5 illustrates a diagrammatic representation of a machine 500 (i.e., medical device locating system) in the form of a computer system within which a set of instructions may be executed for causing the machine to perform any one or more of the methodologies discussed herein. More specifically, FIG. 5 shows a diagrammatic representation of the machine 500 in the example form of a computer system, within which instructions 508 (e.g., software, a program, an application, an applet, an app, or other executable code) for causing the machine 500 to perform any one or more of the methodologies discussed herein may be executed. For example, the instructions 508 may cause the machine 500 to execute instructions 116, routine 200 of FIG. 2, or the like. More generally, the instructions 508 may cause the machine 500 to match IVUS and angiography images and/or image frames as outlined herein.

The instructions 508 transform the general, non-programmed machine 500 into a particular machine 500 programmed to carry out the described and illustrated functions in a specific manner. In alternative embodiments, the machine 500 operates as a standalone device or may be coupled (e.g., networked) to other machines. In a networked deployment, the machine 500 may operate in the capacity of a server machine or a client machine in a server-client network environment, or as a peer machine in a peer-to-peer (or distributed) network environment. The machine 500 may comprise, but not be limited to, a server computer, a client computer, a personal computer (PC), a tablet computer, a laptop computer, a netbook, a set-top box (STB), a PDA, an entertainment media system, a cellular telephone, a smart phone, a mobile device, a wearable device (e.g., a smart watch), a smart home device (e.g., a smart appliance), other smart devices, a web appliance, a network router, a network switch, a network bridge, or any machine capable of executing the instructions 508, sequentially or otherwise, that specify actions to be taken by the machine 500. Further, while only a single machine 500 is illustrated, the term "machine" shall also be taken to include a collection of machines 500 that individually or jointly execute the instructions 508 to perform any one or more of the methodologies discussed herein.

The machine 500 may include processors 502, memory 504, and I/O components 542, which may be configured to communicate with each other such as via a bus 544. In an example embodiment, the processors 502 (e.g., a Central Processing Unit (CPU), a Reduced Instruction Set Computing (RISC) processor, a Complex Instruction Set Computing (CISC) processor, a Graphics Processing Unit (GPU), a Digital Signal Processor (DSP), an ASIC, a Radio-Frequency Integrated Circuit (RFIC), another processor, or any suitable combination thereof) may include, for example, a processor 506 and a processor 510 that may execute the instructions 508. The term "processor" is intended to include multi-core processors that may comprise two or more independent processors (sometimes referred to as "cores") that may execute instructions contemporaneously. Although FIG. 5 shows multiple processors 502, the machine 500 may include a single processor with a single core, a single processor with multiple cores (e.g., a multi-core processor), multiple processors with a single core, multiple processors with multiples cores, or any combination thereof.

The memory 504 may include a main memory 512, a static memory 514, and a storage unit 516, both accessible to the processors 502 such as via the bus 544. The main memory 504, the static memory 514, and storage unit 516 store the instructions 508 embodying any one or more of the methodologies or functions described herein. The instructions 508 may also reside, completely or partially, within the main memory 512, within the static memory 514, within machine-readable medium 518 within the storage unit 516, within at least one of the processors 502 (e.g., within the processor's cache memory), or any suitable combination thereof, during execution thereof by the machine 500.

The I/O components 542 may include a wide variety of components to receive input, provide output, produce output, transmit information, exchange information, capture measurements, and so on. The specific I/O components 542 that are included in a particular machine will depend on the type of machine. For example, portable machines such as mobile phones will likely include a touch input device or other such input mechanisms, while a headless server machine will likely not include such a touch input device. It will be appreciated that the I/O components 542 may include many other components that are not shown in FIG. 5. The I/O components 542 are grouped according to functionality merely for simplifying the following discussion and the grouping is in no way limiting. In various example embodiments, the I/O components 542 may include output components 528 and input components 530. The output components 528 may include visual components (e.g., a display such as a plasma display panel (PDP), a light emitting diode (LED) display, a liquid crystal display (LCD), a projector, or a cathode ray tube (CRT)), acoustic components (e.g., speakers), haptic components (e.g., a vibratory motor, resistance mechanisms), other signal generators, and so forth. The input components 530 may include alphanumeric input components (e.g., a keyboard, a touch screen configured to receive alphanumeric input, a photo-optical keyboard, or other alphanumeric input components), point-based input components (e.g., a mouse, a touchpad, a trackball, a joystick, a motion sensor, or another pointing instrument), tactile input components (e.g., a physical button, a touch screen that provides location and/or force of touches or touch gestures, or other tactile input components), audio input components (e.g., a microphone), and the like.

In further example embodiments, the I/O components 542 may include biometric components 532, motion components 534, environmental components 536, or position components 538, among a wide array of other components. For example, the biometric components 532 may include components to detect expressions (e.g., hand expressions, facial expressions, vocal expressions, body gestures, or eye tracking), measure biosignals (e.g., blood pressure, heart rate, body temperature, perspiration, or brain waves), identify a person (e.g., voice identification, retinal identification, facial identification, fingerprint identification, or electroencephalogram-based identification), and the like. The motion components 534 may include acceleration sensor components (e.g., accelerometer), gravitation sensor components, rotation sensor components (e.g., gyroscope), and so forth. The environmental components 536 may include, for example, illumination sensor components (e.g., photometer), temperature sensor components (e.g., one or more thermometers that detect ambient temperature), humidity sensor components, pressure sensor components (e.g., barometer), acoustic sensor components (e.g., one or more microphones that detect background noise), proximity sensor components (e.g., infrared sensors that detect nearby objects), gas sensors (e.g., gas detection sensors to detection concentrations of hazardous gases for safety or to measure pollutants in the atmosphere), or other components that may provide indications, measurements, or signals corresponding to a surrounding physical environment. The position components 538 may include location sensor components (e.g., a GPS receiver component), altitude sensor components (e.g., altimeters or barometers that detect air pressure from which altitude may be derived), orientation sensor components (e.g., magnetometers), and the like.

Communication may be implemented using a wide variety of technologies. The I/O components 542 may include communication components 540 operable to couple the machine 500 to a network 520 or devices 522 via a coupling 524 and a coupling 526, respectively. For example, the communication components 540 may include a network interface component or another suitable device to interface with a network or multiple networks and/or multiple network devices (not shown). In further examples, the communication components may include wired communication components, wireless communication components, cellular communication components, Near Field Communication (NFC) components, Bluetooth^{®} components (e.g., Bluetooth^{®} Low Energy), Wi-Fi^{®} components, and other communication components to provide communication via other modalities. The devices 522 may be another machine or any of a wide variety of peripheral devices (e.g., a peripheral device coupled via a USB).

Moreover, the communication components may detect identifiers or include components operable to detect identifiers and/or provide identification and/or detection of a medical device within one or more image frames, including but not limited to IVUS image frames and/or OCT image frames. For example, the communication components may include Radio Frequency Identification (RFID) tag reader components, NFC smart tag detection components, optical reader components (e.g., an optical sensor to detect one-dimensional bar codes such as Universal Product Code (UPC) bar code, multi-dimensional bar codes such as Quick Response (QR) code, Aztec code, Data Matrix, Dataglyph, MaxiCode, PDF417, Ultra Code, UCC RSS-2D bar code, and other optical codes), or acoustic detection components (e.g., microphones to identify tagged audio signals). In addition, a variety of information may be derived via the communication components, such as location via Internet Protocol (IP) geolocation, location via Wi-Fi^{®} signal triangulation, location via detecting an NFC beacon signal that may indicate a particular location, and so forth.

The various memories (i.e., memory 504, main memory 512, static memory 514, and/or memory of the processors 502) and/or storage unit 516 may store one or more sets of instructions and data structures (e.g., software) embodying or utilized by any one or more of the methodologies or functions described herein. These instructions (e.g., the instructions 508), when executed by processors 502, cause various operations to implement the disclosed embodiments.

As used herein, the terms "machine-storage medium," "device-storage medium," "computer-storage medium" mean the same thing and may be used interchangeably in this disclosure. The terms refer to a single or multiple storage devices and/or media (e.g., a centralized or distributed database, and/or associated caches and servers) that store executable instructions and/or data. The terms shall accordingly be taken to include, but not be limited to, solid-state memories, and optical and magnetic media, including memory internal or external to processors. Specific examples of machine-storage media, computer-storage media and/or device-storage media include non-volatile memory, including by way of example semiconductor memory devices, e.g., erasable programmable read-only memory (EPROM), electrically erasable programmable read-only memory (EEPROM), FPGA, and flash memory devices; magnetic disks such as internal hard disks and removable disks; magnetooptical disks; and CD-ROM and DVD-ROM disks. The terms "machine-storage media," "computer-storage media," and "device-storage media" specifically exclude carrier waves, modulated data signals, and other such media, at least some of which are covered under the term "signal medium" discussed below.

In various example embodiments, one or more portions of the network in communication with the systems herein may be an ad hoc network, an intranet, an extranet, a VPN, a LAN, a WLAN, a WAN, a WWAN, a MAN, the Internet, a portion of the Internet, a portion of the PSTN, a plain old telephone service (POTS) network, a cellular telephone network, a wireless network, a Wi-Fi^{®} network, another type of network, or a combination of two or more such networks. For example, the connected network or a portion of a network may include a wireless or cellular network, and may be coupled via a Code Division Multiple Access (CDMA) connection, a Global System for Mobile communications (GSM) connection, or another type of cellular or wireless coupling. In this example, the coupling (not shown) may implement any of a variety of types of data transfer technology, such as Single Carrier Radio Transmission Technology (1xRTT), Evolution-Data Optimized (EVDO) technology, General Packet Radio Service (GPRS) technology, Enhanced Data rates for GSM Evolution (EDGE) technology, third Generation Partnership Project (3GPP) including 3G, fourth generation wireless (4G) networks, Universal Mobile Telecommunications System (UMTS), High Speed Packet Access (HSPA), Worldwide Interoperability for Microwave Access (WiMAX), Long Term Evolution (LTE) standard, others defined by various standard-setting organizations, other long range protocols, or other data transfer technology.

The instructions 508 may be transmitted or received over the network (or a portion of a network) using a transmission medium via a network interface device (e.g., a network interface component included in the communication components) and utilizing any one of several well-known transfer protocols (e.g., hypertext transfer protocol (HTTP)). Similarly, the instructions 508 may be transmitted or received using a transmission medium via the couplings disclosed herein (e.g., a peer-to-peer coupling) to the devices disclosed herein. The terms "transmission medium" and "signal medium" mean the same thing and may be used interchangeably in this disclosure. The terms "transmission medium" and "signal medium" shall be taken to include any intangible medium that can store, encoding, or carrying the instructions 508 for execution by the machine 500, and includes digital or analog communications signals or other intangible media to facilitate communication of such software. Hence, the terms "transmission medium" and "signal medium" shall be taken to include any form of modulated data signal, carrier wave, and so forth. The term "modulated data signal" means a signal that has one or more of its characteristics set or changed in such a matter as to encode information in the signal.

The foregoing has broadly outlined the features and technical advantages of the present disclosure such that the following detailed description of the disclosure may be better understood. It is to be appreciated by those skilled in the art that the embodiments disclosed may be readily utilized as a basis for modifying or designing other structures for carrying out the same purposes of the present disclosure. The novel features of the disclosure, both as to its organization and operation, together with further objects and advantages will be better understood from the following description when considered in connection with the accompanying figures. It is to be expressly understood, however, that each of the figures is provided for the purpose of illustration and description and is not intended as a definition of the limits of the present disclosure.

Terms used herein should be accorded their ordinary meaning in the relevant arts, or the meaning indicated by their use in context, but if an express definition is provided, that meaning controls.

Herein, references to "one embodiment" or "an embodiment" do not necessarily refer to the same embodiment, although they may. Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively, unless expressly limited to one or multiple ones. Additionally, the words "herein," "above," "below" and words of similar import, when used in this application, refer to this application as a whole and not to any portions of this application. When the claims use the word "or" in reference to a list of two or more items, that word covers all the following interpretations of the word: any of the items in the list, all the items in the list and any combination of the items in the list, unless expressly limited to one or the other. Any terms not expressly defined herein have their conventional meaning as commonly understood by those having skill in the relevant art(s).

## Claims

1. A computer-implemented method of locating a guide catheter within vasculature, the method comprising:
receiving a series of intravascular image frames captured along a portion of a vessel;
extracting a plurality of longitudinal images from the series of intravascular image frames, wherein each one of the plurality of longitudinal images is sampled at a different angle;
condensing each of the plurality of longitudinal images into corresponding longitudinal intensity values;
averaging the longitudinal intensity values for each of the plurality of longitudinal images;
deriving a series of intensity variation values based in part on the average longitudinal intensity values for each of the plurality of longitudinal images;
identifying a longitudinal location along the portion of the vessel based on the series of intensity variation values; and
outputting an indication of a frame of the series of intravascular image frames corresponding to the longitudinal location.

2. The computer-implemented method of claim 1, wherein extracting the plurality of longitudinal images from the series of intravascular image frames comprises:
sampling the series of intravascular image frames at a plurality of angles; and
generating, for each one of the plurality of angles, a longitudinal image of the plurality of longitudinal images, wherein the longitudinal image is representative of the frames of the plurality of intravascular image frames associated with the one of the plurality of angles.

3. The computer-implemented method of claim 1, wherein condensing each of the plurality of longitudinal images into corresponding longitudinal intensity values comprises summing the intensity at each longitudinal location.

4. The computer-implemented method of claim 1, wherein deriving a series of intensity variation values comprises a sliding window translatable along a longitudinal direction.

5. The computer-implemented method of any one of claims 1-4, wherein the frame of the series of intravascular image frames depicts a guide catheter.

6. The computer-implemented method of any one of claims 1-4, wherein the frame of the series of intravascular image frames indicates a diameter of a guide catheter.

7. The computer-implemented method of any one of claims 1-4, wherein extracting a plurality of longitudinal images comprises extracting four or more longitudinal images.

8. The computer-implemented method of any one of claims 1-4, wherein the series of intravascular image frames are captured using intravascular ultrasound (IVUS) or optical coherence tomography (OCT).

9. An apparatus for an intravascular ultrasound (IVUS) imaging system, comprising:
a display;
an interface configured to couple to an IVUS imaging modality;
a processor coupled to the interface and the display; and
a memory device comprising instructions, which when executed by the processor cause the IVUS imaging system to:
receive a series of intravascular image frames captured along a portion of a vessel;
extract a plurality of longitudinal images from the series of intravascular image frames, wherein each one of the plurality of longitudinal images is sampled at a different angle;
condense each of the plurality of longitudinal images into corresponding longitudinal intensity values;
average the longitudinal intensity values for each of the plurality of longitudinal images;
derive a series of intensity variation values based in part on the average longitudinal intensity values for each of the plurality of longitudinal images;
identify a longitudinal location along the portion of the vessel based on the series of intensity variation values; and
output an indication of a frame of the series of intravascular image frames corresponding to the longitudinal location.

10. The apparatus of claim 9, wherein the extracted plurality of longitudinal images from the series of intravascular image frames comprises:
a series of intravascular image frames sampled at a plurality of angles; and
for each one of the plurality of angles, a generated longitudinal image of the plurality of longitudinal images, wherein the longitudinal image is representative of the frames of the plurality of intravascular image frames associated with the one of the plurality of angles.

11. The apparatus of claim 9, wherein each of the plurality of images are condensed into corresponding longitudinal intensity values by summing the intensity at each longitudinal location.

12. The apparatus of any one of claims 9-11, wherein the frame of the series of intravascular image frames depicts a guide catheter.

13. The apparatus of any one of claims 9-11, wherein the series of intravascular image frames are captured using intravascular ultrasound (IVUS) or optical coherence tomography (OCT).

14. A non-transitory computer-readable storage medium, the computer-readable storage medium including instructions that when executed by a computer, cause the computer to:
receive a series of intravascular image frames captured along a portion of a vessel;
extract a plurality of longitudinal images from the series of intravascular image frames, wherein each one of the plurality of longitudinal images is sampled at a different angle;
condense each of the plurality of longitudinal images into corresponding longitudinal intensity values;
average the longitudinal intensity values for each of the plurality of longitudinal images;
derive a series of intensity variation values based in part on the average longitudinal intensity values for each of the plurality of longitudinal images;
identify a longitudinal location along the portion of the vessel based on the series of intensity variation values; and
output an indication of a frame of the series of intravascular image frames corresponding to the longitudinal location.

15. The non-transitory computer-readable storage medium of claim 14, wherein the frame of the series of intravascular image frames depicts a guide catheter.
